# EUROPEAN PATENT APPLICATION

(11) **EP 0 661 057 A1**
(43) Date of publication of application: **05.07.1995**
(21) Application number: 94917786.9
(22) Date of filing: 08.06.1994
(51) Int. Cl.: A61K 38/00

(54) **HEMATOPOIETIC CELL PROLIFERATION ACCELERATOR**

(30) Priority: 08.06.1993 JP 137963/93; 09.05.1994 JP 95059/94
(71) Applicant: Ajinomoto Co., Inc., Tokyo 104 (JP)
(72) Inventor: OKANO, Akira, Central Research Laboratories,, Kawasaki-shi Kanagawa-ken 210 (JP); SUZUKI, Hideki, Central Research Laboratories,, Kawasaki-shi Kanagawa-ken 210 (JP)
(74) Representative: Strehl Schübel-Hopf Groening & Partner
(86) International application number: JP9400927
(87) International publication number: WO9428919

(57) **Abstract**

A hematopoietic cell proliferation accelerator comprising (1) human BCDF or (2) both of human BCDF and human G-CSF as the active ingredient. It can proliferate leukocytes, blood platelet, hematopoietic precursor cells and multipotential hematopoietic stem cells, and efficacious in treating or preventing complications accompanying hematopenia, such as infectious diseases or hemorrhage, and particularly efficacious for peripheral blood stem cell transplantation.

## Description

### Technical Field

The present invention relates to a hemopoietic cell proliferation(expansion and/or mobilization)-promoting agent. More specifically, the present invention relates to a hemopoietic cell proliferation-promoting agent comprising (1) human BCDF (B cell differentiation factor) or (2) both human BCDF and human G-CSF (human granulocyte-colony stimulating factor) as effective components.

### Background Art

The hemocytes in the blood are continuously produced from pluripotent hemopoietic stem cells present in the bone marrow through hemopoietic progenitor cells. In practical clinic, most of the tumors such as leukemia have been treated while making use of chemotherapeutic and radiotherapeutic techniques. However, these therapeutic methods inhibit the proliferation of not only tumor cells but also normal bone marrow cells and accordingly, result in a decrease of the hemocytes in the blood. More specifically, these therapeutic methods lead to decreases of, in particular, leukocytes which play an important role in the defence of living bodies against foreign attacks and platelets which play an important role in hemostasis and accordingly, such living bodies are susceptible to receive attacks of microorganisms. As a result, the living bodies suffer from so-called oppotunistic infections and severe infectious diseases or cause internal hemorrhage within the skull.

As has been discussed above, there has been known that the treatment of tumors by the chemotherapeutic and radiotherapeutic techniques is accompanied by leukocytopenia and thrombopenia. As a recent attempt to solve the problem, human G-CSF has been administered to patients to thus induce an increase in the number of leukocytes. However, there has been desired for the development of a method for further improvement in the proliferation ability of leukocytes.

Moreover, it has been known that the human G-CSF mainly serves to simply increase the number of leukocytes (especially neutrophil) and is poor in the effect of increasing platelet-number. For this reason, there has been desired for the development of an agent capable of increasing the nubmer of not only leukocytes, but also other hemocytes such as platelets.

Therapeutic methods which make use of bone marrow transplantation and peripheral blood stem cell transplantation techniques have been known as a therapy for treating hemocytopenia associaed with the treatments of tumors.

According to the bone marrow transplantation technique, normal pluripotent hemopoietic stem cells present in donor's bone marrow are collected and then transplanted to patients(recipients) after killing all of the tumor cells and the normal hemopoietic cells of the patients by strong chemotherapy and radiotherapy. Alternatively, it has also been known that cells collected from a patient per se (self) and stored prior to the foregoing strong chemotherapy and radiotherapy are transplanted after the therapeutic treatments. The autologus stem cell transplantation has such a merit that there is not observed any graft-versus-host disease response, but the radiotherapy or the like sometimes makes it difficult to collect a sufficient amount of hemopoietic stem cells from the ilium portion of patients. Moreover, the infiltration of tumor into the bone marrow further makes it difficult to collect hemopoietic stem cells from a patient per se. In addition, the bone marrow transplantation technique requires application of general anesthesia to a donor (or in some cases, patients per se) when collecting hemopoietic stem cells and accordingly, the donor is significantly influenced by this technique.

On the other hand, the peripheral blood stem cell transplantation technique comprises the steps of collecting and storing peripheral blood stem cells and then transplanting the stem cells to a patient for hematological reconstruction after the destructive therapy (such as chemotherapy and radiotherapy). The peripheral blood stem cell transplantation is a therapeutic technique developed on the basis of the knowledges that the pluripotent hemopoietic stem cells are present not only in the bone marrow, but also in the peripheral blood although the population thereof is low and that the peripheral blood stem cells show a significant, but transient increase during the hemopoietic recovery after the myelosuppressive chemotherapy. This technique is advantageous in that it does not require the application of general anesthesia unlike the bone marrow transplantation technique. However, the pluripotent hemopoietic cells are present in the peripheral blood in a very small number and accordingly, the cells must be isolated and collected over a plurality of times in order to obtain a sufficient number of the hemopoietic stem cells.

Incidentally, the human BCDF shows an activity of differentiating mature B cells into antibody-producing cells, is a factor proposed to call IL-6 (interleukin-6). The protein structure, DNA sequence and amino acid sequence of the human BCDF are already determined (see Japanese Un-examined Patent Publication (hereunder referred to as "J.P. KOKAI") Nos. Sho 61-115025, Sho 63-42688 and Sho 63-56291) and methods for producing the human BCDF using Escherichia coli are already disclosed (J.P. KOKAI Nos. Sho 63-157996 and Hei 3-244391). Moreover, further investigations make it clear that the human BCDF can be used as an effective immunotherapeutic agent for treating infectious diseases and tumors (J.P. KOKAI No. Sho 64-63524), that it can serve as a supporting agent for the bone marrow transplantation (J.P. KOKAI No. Hei 2-76820), that it can serve as a supporting agent for tumor-suppressive therapy (J.P. KOKAI No. Hei 3-31215) and that it can serve as an agent for treating thrombocytopenia (J.P. KOKAI No. Hei 3-101624). However, there has not yet been submitted any report concerning influences of in vivo-administration of the human BCDF on the peripheral blood hemopoietic progenitor cells and the pluripotent hemopoietic stem cells.

On the other hand, the human G-CSF is known to be a factor which serves to differentiate myeloid lineage progenitor cells into neutrophils and the DNA and amino acid sequences thereof are already determined (Nature, 1986, 319, p. 415) and it has practically been put into clinical use, as a neutrophil proliferation-promoting factor, in treatments of leukocytopenia observed during treatments of, for instance, tumors. However, it has not yet been reported that a medicine comprising both of these human BCDF and human G-CSF is effective as an agent for promoting the proliferation of hemopoietic cells in the peripheral blood.

### Disclosure of the Invention

An object of the present invention is to provide a novel hemopoietic cell proliferation-promoting agent capable of increasing hemopoietic cells in the peripheral blood, i.e., leukocytes, platelets, hemopoietic progenitor cells and pluripotent hemopoietic stem cells.

The inventors of this invention have conducted various studies to accomplish the foregoing object, have found out that a hemopoietic cell proliferation-promoting agent which comprises (1) human BCDF or (2) both human BCDF and human G-CSF as effective components permits (a) increases of leukocytes and platelets present in the peripheral blood; (b) increases of leukocytes and platelets present in the peripheral blood during treating a patient by radiotherapy or chemotherapy; (c) increases of hemopoietic progenitor cells and pluripotent hemopoietic stem cells present in the peripheral blood; (d) an increase of hemopoietic progenitor cells present in the peripheral blood during treating a patient by radiotherapy or chemotherapy; and (e) an increase of hemopoietic cells in the peripheral blood and that the hemopoietic cells present in the peripheral blood thus proliferated can be used in the peripheral blood stem cell transplantation, and thus have completed the present invention.

Thus, the present invention relates to a hemopoietic cell proliferation-promoting agent which comprises (1) human BCDF or (2) both human BCDF and human G-CSF as effective components.

### Best Mode for Carrying Out the Invention

The present invention will hereunder be described in detail.

The human BCDF used in the present invention may be those having any structure insofar as they have a human BCDF activity. Among these, preferred are those having amino acid sequences as disclosed in Nature, 1986, 324, p. 73, in other words, a peptide (hereunder referred to as "natural human BCDF; the amino acid sequence thereof will be detailed in the Sequence Listing given later as Sequence No. 1) having a Pro residue at the N-terminal, a Met residue at the C-teminal and comprising 184 amino acid residues in all and a peptide (hereunder referred to as "human Ala-BCDF"; the amino acid sequence thereof will also be detailed in the Sequence Listing given later as Sequence No. 2) having the same amino acid sequence of the natural human BCDF except that an Ala residue is linked to the N-terminal on the upstream side of the Pro residue.

Saying over again, the human BCDF used in the present invention must not have a structure identical to that of the natural human BCDF or the human Ala-BCDF. More specifically, the human BCDF used herein may be those having the structure of the natural human BCDF to the N-and/or C-terminals of which one or a plurality of amino acid residues are added or having the structure of the natural human BCDF in which one or plurality of amino acid residues are replaced with other amino acid residues insofar as they exhibit the human BCDF activity. Moreover, these human BCDF's may be not only non-glycosylated BCDF's produced by Escherichia coli, but also glycosylated BCDF's produced by eucaryotic cells such as CHO cells (Chinese hamster ovary cells).

The human G-CSF usable herein may be, for instance, a polypeptide having 177 amino acid residues as disclosed in Nature, 1986, 319, p. 415 (the amino acid sequence thereof is detailed in the Sequence Listing given later as Sequence No. 3) or a polypeptide having 174 amino acid residues as disclosed in Science, 1986, 232, p. 61 (the amino acid sequence thereof is detailed in the Sequence Listing given later as Sequence No. 4). Moreover, it is also possible to use polypeptides which are replaced with amino acid residues as disclosed in Biochem. Biophys. Res. Commun., 1989, 159, p. 103. In addition, the human G-CSF's may be not only non-glycosylated G-CSF's produced by Escherichia coli, but also glycosylated G-CSF's produced by eucaryotic cells such as CHO cells.

The amount of (1) the human BCDF or (2) the combination of the human BCDF and the human G-CSF used in the hemopoietic cell proliferation-promoting agent of the present invention in general ranges from 0.0001 to 100% by weight and preferably 0.1 to 1.0% by weight.

In the hemopoietic cell proliferation-promoting agent of the present invention, relative amounts of the human BCDF and the human G-CSF are not restricted to specific ranges, but the amount of the human G-CSF in general ranges from 0.0001 to 200000 parts by weight and preferably 0.1 to 100 parts by weight per 100 parts by weight of the human BCDF.

The hemopoietic cell proliferation-promoting agent of the present invention comprising (1) the human BCDF or (2) the combination of the human BCDF and the human G-CSF as effective components may further comprise a stabilizer such as serum albumin and/or an excipient such as mannitol.

Moreover, the hemopoietic cell proliferation-promoting agent of the present invention may comprise, in addition to the human BCDF and the human G-CSF, at least one auxiliary agent selected from the group consisting of, for instance, cytokines other than the human G-CSF such as human IL-1, human IL-3, human IL-11, human SCF (human stem cell factor), human LIF (human leukemia inhibitory factor), human EPO (human erythropoietin), human GM-CSF (human granulocyte/macrophage-colony-stimulating factor) and human M-CSF (human macrophage-colony-stimulating factor). If the hemopoietic cell proliferation-promoting agent comprises these auxiliary agents, i.e., human IL-1, human IL-3, human IL-11, human SCF, human ILF, human EPO, human GM-CSF and human M-CSF for the purpose of accelerating the hemopoietic function, the resulting hemopoietic cell proliferation-promoting agent exhibits a synergistically improved effect of increasing hemopoietic cells.

Cytokines which is known to use a receptor identical to that of the human BCDF such as human IL-11, human LIF, human CNTF (human ciliary neurotrophic factor) and human oncostatin M would also be used alone or in combination instead of the human BCDF.

In this respect, the amount of these auxiliary agents to be incorporated into the hemopoietic cell proliferation-promoting agent is not limited to a particular range, but may be added in an amount ranging from 0.0001 to 200000 parts by weight per 100 parts by weight of human BCDF or human G-CSF. Saying over again, the added amount of these auxiliary agents is never limited to the range defined above and may appropriately be determined or selected depending on, for instance, the symptomes and age of a patient.

It is not necessary that auxiliary agents such as human G-CSF or human IL-1 are administered to a patient simultaneous with the medicine comprising, as effective components, (1) the human BCDF or (2) a combination of the human BCDF and the human G-CSF by, for instance, incorporating them into the medicine. More specifically, it is possible to administer these auxiliary agents to a patient at an appropriate instance prior to or after the administration of the medicine comprising, as effective components, (1) the human BCDF or (2) a combination of the human BCDF and the human G-CSF.

The dose of the hemopoietic cell proliferation-promoting agent of the present invention is not particularly restricted, but the dose thereof preferably ranges from 0.1 to 1000 µg/kg/day for the human BCDF and 0.1 to 1000 µg/kg/day for the human G-CSF.

The hemopoietic cell proliferation-promoting agent of the present invention may be administered through any method and route such as intravenous injection, intramuscular injection and subcutaneous injection.

Moreover, the time required for administration of unit dosage is not particularly limited as well and unit dosage thereof may be administered within a short period of time or may be subjected to sustained administration over not less than 24 hours.

The term for administration of the hemopoietic cell proliferation-promoting agent of the present invention is not limited to a specific range, but preferably ranges from 1 to 14 days and more preferably 3 to 14 days.

The dosage time of the hemopoietic cell proliferation-promoting agent of the present invention is not limited to a specific range as well. More specifically, the dosage time per day is not particularly limited and the agent is preferably administered 1 to 3 times per day. In addition, it may be administered everyday or at intervals of not less than one day. In other words, the agent can be administered according to any schedule.

The hemopoietic cell proliferation-promoting agent of the present invention may of course be used in combination with other chemotherapeutic agents (such as antineoplastic agents, antiviral agents and antibiotics) and/or immunotherapeutic agents (such as immunopotentiators and immunosuppressive agents).

The hemopoietic cell proliferation-promoting agent of the present invention which comprises (1) the human BCDF or (2) a combination of the human BCDF and the human G-CSF may principally be used according to the following two methods.

In one method, the hemopoietic cell proliferation-promoting agent is used when the number of hemocytes is reduced during treating a patient suffering from, for instance, tumors in order to promote the recovery thereof. More specifically, the agent can be administered to any patient who has been treated by chemotherapy or radiotherapy or subjected to hemopoietic stem cell trasnsplantation in the bone marrow or the peripheral blood and in which a decrease in the number of hemocytes would accordingly be expected. The agent may be administered to a patient prior to or after the treatment in case of chemotherapy or radiotherapy. After treatments by, for instance, radiotherapy, the agent is desirably administered immediately after the treatments, but it is also possible to use the agent after the number of hemocytes is reduced. Moreover, the agent is desirably administered to a patient immediately after the treatment in case of hemopoietic stem cell transplantation.

In the other method, the agent is administered when collecting peripheral blood progenitor cells or peripheral blood stem cells for the purpose of increasing the population of the stem cells. The peripheral blood stem cells are collected from either a normal person or a patient per se suffering from, for instance, a tumor. If the stem cells are collected from a normal person, the agent may be administered thereto at any instance prior to the collection, while if the agent is administered to a patient suffering from, for instance, a tumor, they are preferably collected from the patient during recovery phase from the myellosuppression observed after treatments(such as chemotherapy and/or radiotherapy), but it may be administered prior to treatments or immediately after the treatments. The agent may be administered to both normal persons and patients under the conditions for administration discussed above.

The hemopoietic cell proliferation-promoting agent according to the present invention which comprises (1) the human BCDF or (2) a combination of the human BCDF and the human G-CSF as effective components exhibits an effect of promoting hematological reconstruction of a patient and is accordingly effective for treatments and/or prevention of a reduction in the number of hemocytes accompanied by complications such as infectious deseases and bleeding. The hemopoietic cell proliferation-promoting agent of the present invention also has an effect of increasing the population of peripheral blood stem cells and therefore, the agent is likewise effectively used for collecting a sufficient number of peripheral blood stem cells required for the transplantation thereof.

The following Examples are given in order to more specifically explain the present invention, but the present invention is by no means limited to these specific Examples.

### Example 1

### Increases in Numbers of Leukocytes and Platelets Through In Vivo Administration of (1) Human BCDF or (2) Both Human BCDF and Human G-CSF

Human BCDF (140 µg; 10 µg/animal/day × 14 days) and/or human G-CSF (5.04 µg; 0.36 µg/animal × 14 days) were charged to osmotic pumps available from Alza Company and the pumps were subcutaneously transplanted in C57BL/6 mice (8 female animals of 6 to 9-week-old) for continuous administration. As a control, HSA (human serum albumin) was administered to mice in an amount identical to that of the human BCDF. After 14 days, the peripheral blood was collected from the heart of each mouse and the number of hemocytes (leukocytes and platelets) present in the peripheral blood was determined using an automatic blood cell counter (available from Toa Medical Electronic Co., Ltd.). In this Example, there were used so-called natural human BCDF produced by Escherichia coli and having the amino acid sequence detailed in Sequence Listing: Sequence No. 1 given later as the human BCDF and a glycosylated natural human G-CSF (trade name: Lenograstim available from Chugai Pharmaceutical Co., Ltd.) produced by CHO cells and having the amino acid sequence detailed in Sequence Listing: Sequence No. 4 given later as the human G-CSF.

The data listed in Table 1 given below indicate that the numbers of both leukocytes and platelets were significantly increased, as compared with the control, in response to the administration of the human BCDF.

Moreover, the number of leukocytes was significantly increased through the simultaneous administration of the human BCDF and the human G-CSF as compared with not only the control, but also those observed for the administration of the human BCDF alone or the human G-CSF alone. The differential counts of leukocytes make it clear that neutrophils were mainly increased, but the number of, for instance, lymphocytes was also increased. The number of platelets was increased as compared with the control, but there was not observed any difference between the simultaneous administration and the administration of the human BCDF alone in number of platelets.

**Table 1**

| | No. of Leukocytes (X 10²/µl) | No. of Platelets (X 10⁴/µl) |
|---|---|---|
| Normal Mouse | 29.4±9.0 | 85.4±6.5 |

| Mouse Treated | | |
|---|---|---|
| Control | 38.9±8.1 | 106.1±11.2 |
| Administration of Human BCDF | 63.0±21.5* | 147.7±17.9* |
| Administration of Human G-CSF | 80.5±24.9* | 95.2±18.4 |
| Administration of Human BCDF + Human G-CSF | 378.8±305.1*§ | 149.1±11.8* |
| Each data represented means ± standard deviation of 8 mice. | | |

| | | |
|---|---|---|
| *: This means that a significant difference is observed between each corresponding value and the control at a risk rate of 5%. | | |
| § : This means that a significant difference is observed at a risk rate of 5% with respect to both the administration of the human BCDF alone and the administration of the human G-CSF alone. | | |

### Example 2

### Recovery in Numbers of Leukocytes and Platelets Through In Vivo Administration of (1) Human BCDF or (2) Human BCDF and Human G-CSF, in X-Ray Irradiation-Induced Cytopenia Model

C57BL/6 mice (4 female animals of 10-week-old) were irradiated with X-rays at a dose of 4.0 Gy over the entire bodies thereof. One day after the X-ray irradiation, human BCDF (140 µg; 10 µg/animal × 14 days) and/or human G-CSF (5.04 µg; 0.36 µg/animal × 14 days) were charged to osmotic pumps available from Alza Company and the pumps were subcutaneously transplanted in these animals for continuous administration. As a control, HSA (human serum albumin) was administered to mice in an amount identical to that of the human BCDF. Fifteen days after the X-ray irradiation (i.e., 14 days after the initiation of the administration), the peripheral blood was collected from the heart of each mouse and the number of hemocytes (leukocytes and platelets) present in the peripheral blood was determined using an automatic blood cell counter (available from Toa Medical Electronic Co., Ltd.). The human BCDF and the human G-CSF used in this Example were the same as those used in Example 1.

As shown in Table 2 given below, the numbers of leukocytes and platelets reduced due to the X-rays irradiation were both significantly recovered and increased through the administration of the human BCDF as compared with the control. Moreover, the number of leukocytes was quite significantly increased through the simultaneous administration of the human BCDF and the human G-CSF as compared with not only the control, but also those observed for the administration of the human BCDF alone and the human G-CSF alone. The differential counts of leukocytes make it clear that neutrophils were mainly increased, but the number of, for instance, lymphocytes was also increased. The number of platelets was increased as compared with the control, but there was not observed any difference between the simultaneous administration and the administration of the human BCDF alone in number of platelets.

**Table 2**

| | No. of Leukocytes (X 10²/µl) | No. of Platelets (X 10⁴/µl) |
|---|---|---|
| Normal Mouse (free of X-ray Irradiation) | 57.0±21.2 | 101.1±9.6 |

| Mouse Treated (Irradiated with X-Rays) | | |
|---|---|---|
| Control | 22.3±4.3 | 89.2±4.0 |
| Administration of Human BCDF | 71.8±15.3* | 141.0±7.1* |
| Administration of Human G-CSF | 221.5±119.7* | 84.9±11.0 |
| Administration of Human BCDF + Human G-CSF | 2216.0±30.7* § | 135.4±10.1* |
| Each data represented means ± standard deviation of 4 mice. | | |

| | | |
|---|---|---|
| *: This means that a significant difference is observed between each corresponding value and the control at a risk rate of 5%. | | |
| § : This means that a significant difference is observed at a risk rate of 5% with respect to both the administration of the human BCDF alone and the administration of the human G-CSF alone. | | |

### Example 3

### Recovery in Numbers of Leukocytes and Platelets Through In Vivo Administration of (1) Human BCDF or (2) Human BCDF and Human G-CSF, in Chemotherapeutic Agent Administration-Induced Cytopenia Model

Carboplatin (CBDCA) was administered to C57BL/6 mice (4 female animals of 10-week-old) at a dose of 50 mg/kg every other day over three times through the tail vein. One day after the final administration, human BCDF and/or human G-CSF were subcutaneously administered through sustained administration in the same manner used in Example 2. As a control, HSA (human serum albumin) was administered to mice in an amount identical to that of the human BCDF. Fourteen days after the initiation of the administration, the peripheral blood was collected from the heart of each mouse and the number of hemocytes (leukocytes and platelets) present in the peripheral blood was determined. The human BCDF and the human G-CSF used in this Example were the same as those used in Example 1.

As shown in Table 3 given below, the numbers of leukocytes and platelets reduced due to the administration of the chemotherapeutic agent were both significantly recovered and increased through the administration of the human BCDF as compared with the control. Moreover, the number of leukocytes was quite significantly increased through the simultaneous administration of the human BCDF and the human G-CSF as compared with not only the control, but also those observed for the administration of the human BCDF alone and the human G-CSF alone. The differential count of leukocytes makes it clear that neutrophils were mainly increased, but the number of, for instance, lymphocytes was also increased. The number of platelets was increased as compared with the control, but there was not observed any difference between the simultaneous administration and the administration of the human BCDF alone in number of platelets.

**Table 3**

| | No. of Leukocytes (X 10²/µl) | No. of Platelets (X 10⁴/µl) |
|---|---|---|
| Normal Mouse (free of administration of chemotherapeutic agent) | 43.5± 6.4 | 99.6±6.4 |

| Mouse Treated (treated with chemotherapeutic agent) | | |
|---|---|---|
| Control | 26.5±9.6 | 67.5±18.7 |
| Administration of Human BCDF | 47.0± 4.7* | 115.2±29.9* |
| Administration of Human G-CSF | 88.5±15.3* | 83.3±14.1 |
| Administration of Human BCDF + Human G-CSF | 902.8±446.2*§ | 113.8±27.8* |
| Each data represented means ± standard deviation of 4 mice. | | |

| | | |
|---|---|---|
| *: This means that a significant difference is observed between each corresponding value and the control at a risk rate of 5%. | | |
| § : This means that a significant difference is observed at a risk rate of 5% with respect to both the administration of the human BCDF alone and the administration of the human G-CSF alone. | | |

The following Examples 4 to 8 relate to increases in the hemopoietic progenitor cells and the pluripotent hemopoietic stem cells present in the peripheral blood, which are induced by the in vivo administration of (1) human BCDF or (2) a combination of human BCDF and human G-CSF.

### Example 4

Human BCDF (140 µg; 10 µg/animal × 14 days) and/or human G-CSF (5.04 µg; 0.36 µg/animal × 14 days) were charged to osmotic pumps available from Alza Company and the pumps were subcutaneously transplanted in C57BL/6 mice (4 female animals of 6-week-old) for continuous administration. As a control, HSA (human serum albumin) was administered to mice in an amount identical to that of the human BCDF. Fourteen days after the initiation of the administration, the peripheral blood was collected, under sterilized conditions, from the heart of each mouse. The human BCDF and the human G-CSF used in this Example were the same as those used in Example 1.

The blood collected from each group was diluted with the same volume of a heparin-containing IMDM culture medium and pooled. The pooled blood sample was layered on Lympholite M (available from Sedalane Company) and centrifuged to give a mononuclear cell layer free of red blood cells. The mononuclear cells thus obtained were diluted to an appropriate concentration, then cultured, for 7 days, in 1 ml of a 0.8% methylcellulose semi-solid medium containing 200 U/ml of mouse IL-3 and 2 U/ml of erythropoietin, followed by determination of the number of myeloid cell clusters (colonies) formed which was defined to be that of myeloid progenitor cells (CFU-GM). Moreover, the number of colonies containing both myeloid and erythroid and formed after the cultivation over 15 days was determined and defined to be that of pluripotent hemopoietic stem cells (CFU-GEM). Then the numbers of CFU-GM and CFU-GEM per 1 ml of the original peripheral blood were calculated from these results while taking into consideration of the rate of dilution.

As shown in Table 4 given below, the number of CFU-GM's was significantly increased as compared with the control through the administration of (1) the human BCDF and that of CFU-GEM's also had a tendency to increase. Moreover, the numbers of CFU-GM's and CFU-GEM's were both quite significantly increased through the simultaneous administration of the human BCDF and the human G-CSF as compared with not only the control, but also those observed for the administration of the human BCDF alone and the human G-CSF alone.

**Table 4**

| | No. of CFU-GM/ml Peripheral Blood | No. of CFU-GEM/ml Peripheral Blood |
|---|---|---|
| Normal Mouse | 33 ± 32 | 3.3 ± 5.8 |

| Mouse Treated | | |
|---|---|---|
| Control | 80 ± 20 | 6.7 ±11.6 |
| Administration of Human BCDF | 947 ±110* | 40.0 ±26.5 |
| Administration of Human G-CSF | 577 ± 51* | 40.0 ±0.0* |
| Administration of Human BCDF + Human G-CSF | 10233 ±153*§ | 466.7±152.8*§ |
| Each data represented means ± standard deviation of 3 dishes × rate of dilution. | | |

| | | |
|---|---|---|
| *: This means that a significant difference is observed between each corresponding value and the control at a risk rate of 5%. | | |
| § : This means that a significant difference is observed at a risk rate of 5% with respect to both the administration of the human BCDF alone and the administration of the human G-CSF alone. | | |

Separately, the peripheral blood sample (0.01 ml each) derived from each animal was inspected for the presence of CFU-GEM, but no CFU-GEM was detected in neither the normal mice nor the control mice, while CFU-GEM was detected in 25% of the peripheral blood derived from (1) the human BCDF-administered animal group and in 75% of the peripheral blood derived from (2) the human BCDF and human G-CSF-administered animal group.

### Example 5

Separate experiments were carried out to investigate the behavior of erythroblastic hemopoietic progenitor cells. In the same manner used in the foregoing experiments, human BCDF and human G-CSF were subcutaneously administered through sustained administration and 14 days after the initiation of the administration, the peripheral blood was collected, under sterilized conditions, from the heart of each mouse. The mononuclear cells thus obtained were then cultured, for 3 days, in 1 ml of a methylcellulose semi-solid medium, followed by determination of the number of erythroid clusters (colonies) formed which was defined to be that of CFU-E's. The human BCDF and the human G-CSF used in this Example were the same as those used in the foregoing experiments. Moreover, the erythroid clusters (bursts) formed during the cultivation over 10 days were determined and defined to be BFU-E. Then the numbers of CFU-E's and BFU-E's per 1 ml of the original peripheral blood were calculated from these results while taking into consideration of the rate of dilution.

As shown in Table 5 given below, the results of the CFU-E and BFU-E determination were similar to those of the CFU-GM and CFU-GEM determination. More specifically, CFU-E was significantly increased as compared with the control through the administration of (1) the human BCDF and that of BFU-E also had a tendency to increase. Moreover, the number of CFU-E was quite significantly increased through the simultaneous administration of (2) a combination of the human BCDF and the human G-CSF as compared with not only the control, but also those observed for the administration of the human BCDF alone and the human G-CSF alone and that of BFU-E also had a tendency to increase.

**Table 5**

| | No. of CFU-E/ml Peripheral Blood | No. of BFU-E/ml Peripheral Blood |
|---|---|---|
| Normal Mouse | 53 ± 61 | < 3.3 |

| Mouse Treated | | |
|---|---|---|
| Control | 53 ± 23 | < 3.3 |
| Administration of Human BCDF | 2219 ±961* | 6.7 ± 5.8 |
| Administration of Human G-CSF | 6101 ±2450* | 30.0 ±10.0* |
| Administration of Human BCDF + Human G-CSF | 76373 ±3911* § | 400.0 ±100.0* |
| Each data represented means ± standard deviation of 3 dishes × rate of dilution. | | |

| | | |
|---|---|---|
| *: This means that a significant difference is observed between each corresponding value and the control at a risk rate of 5%. | | |
| § : This means that a significant difference is observed at a risk rate of 5% with respect to both the administration of the human BCDF alone and the administration of the human G-CSF alone. | | |

### Example 6

Separate experiments were carried out to investigate the behavior of megakaryocytic progenitor cells (CFU-Mk) and hemopoietic stem cells (CFU-S). In the same manner used in Example 5, human BCDF and human G-CSF were subcutaneously administered to mice through sustained administration and 14 days after the initiation of the administration, the peripheral blood was collected, under sterilized conditions, from the heart of each mouse. The human BCDF and the human G-CSF used in this Example were the same as those used in the foregoing experiments. As a control, HSA (human serum albumin) was administered to mice in an amount identical to that of the human BCDF. The mononuclear cells thus obtained were diluted to an appropriate concentration, then cultured, for 6 days, in 0.4 ml of fibrin clot containing 200 U/ml of mouse IL-3, followed by determination of the number of acetylcholine esterase-positive cell clusters (colonies) formed which was defined to be that of CFU-Mk's. Moreover, the mononuclear cells appropriately diluted were transplanted to syngeneic mice which had been irradiated with X-rays at a dose of 8.0 Gy and after 12 days, the number of colonies formed in the spleen was counted and defined to be that of CFU-S. Then the numbers of CFU-Mk's and CFU-S's per 1 ml of the original peripheral blood were calculated from these results while taking into consideration of the rate of dilution.

As shown in Table 6 given below, the results of the CFU-Mk and CFU-S determination were similar to those of the CFU-GM determination. More specifically, they were significantly increased as compared with the control through the administration of (1) the human BCDF. Moreover, they were quite significantly increased through the simultaneous administration of (2) a combination of the human BCDF and the human G-CSF as compared with not only the control, but also those observed for the administration of the human BCDF alone and the human G-CSF alone.

**Table 6**

| | No. of CFU-Mk/ml Peripheral Blood | No. of CFU-S/ml Peripheral Blood |
|---|---|---|
| Normal Mouse | 5 ± 10 | 6 ±4 |

| Mouse Treated | | |
|---|---|---|
| Control | 5 ± 6 | 9 ±1 |
| Administration of Human BCDF | 55 ±13* | 83 ±20* |
| Administration of Human G-CSF | 213 ±79* | 108 ±31* |
| Administration of Human BCDF + Human G-CSF | 925 ±222*§ | 1240 ±277*§ |
| Each data represented means ± standard deviation of 3 dishes × rate of dilution for CFU-Mk and means ± standard deviation 0+4 spleens × rate of dilution for CFU-S. | | |

| | | |
|---|---|---|
| *: This means that a significant difference is observed between each corresponding value and the control at a risk rate of 5%. | | |
| § : This means that a significant difference is observed at a risk rate of 5% with respect to both the administration of the human BCDF alone and the administration of the human G-CSF alone. | | |

### Example 7

Furthermore, the number of the hemopoietic progenitor cells present in the peripheral blood in X-ray irradiation-induced cytopenia model was examined by in vivo-administering (1) human BCDF or (2) human BCDF and human G-CSF.

In the same manner used in Example 2, C57BL/6 mice (4 female animals of 8-week-old) were irradiated with X-rays at a dose of 4.0 Gy over the entire bodies thereof. One day after the X-ray irradiation, human BCDF and/or human G-CSF were subcutaneously administered to these animals through sustained administration. As a control, HSA (human serum albumin) was administered to mice in an amount identical to that of the human BCDF. Fifteen days after the X-ray irradiation (i.e., 14 days after the initiation of the administration), the peripheral blood was collected from the heart of each mouse under sterilized conditions. Then mononuclear cells were isolated from the peripheral blood and CFU-GM thereof was determined. The human BCDF and the human G-CSF used in this Example were the same as those used in the foregoing experiments.

As shown in Table 7 given below, the number of CFU-GM was significantly increased through the administration of (1) the human BCDF as compared with the control. Moreover, the number of CFU-GM was quite significantly increased through the simultaneous administration of (2) the human BCDF and the human G-CSF as compared with not only the control, but also those observed for the administration of the human BCDF alone and the human G-CSF alone.

**Table 7**

| | No. of CFU-GM/ml Peripheral Blood |
|---|---|
| Normal Mouse (free of X-ray irradiation) | 47 ± 21 |

| Mouse Treated (treated by X-ray irradiation) | |
|---|---|
| Control | 73 ± 35 |
| Administration of Human BCDF | 3400 ±458* |
| Administration of Human G-CSF | 4033 ±586* |
| Administration of Human BCDF + Human G-CSF | 49333 ±19858*§ |
| Each data represented means ± standard deviation of 3 dishes × rate of dilution. | |

| | |
|---|---|
| *: This means that a significant difference is observed between each corresponding value and the control at a risk rate of 5%. | |
| § : This means that a significant difference is observed at a risk rate of 5% with respect to both the administration of the human BCDF alone and the administration of the human G-CSF alone. | |

### Example 8

In this Example, there was investigated the behavior in the number of hemopoietic progenitor cells present in the peripheral blood induced by in vivo-administration of (1) human BCDF or (2) human BCDF and human G-CSF, in chemotherapeutic agent administration-induced cytopenia model.

In the same manner used in Example 3, a chemotherapeutic agent (CBDCA) was administered to C57BL/6 mice (4 female animals of 10-week-old) every other day over three times through the tail vein. One day after the final administration, human BCDF and/or human G-CSF were subcutaneously administered through sustained administration. As a control, HSA (human serum albumin) was administered to mice in an amount identical to that of the human BCDF. Fourteen days after the initiation of the administration, the peripheral blood was collected from the heart of each mouse under sterilized conditions. Then mononuclear cells were isolated from the peripheral blood and CFU-GM thereof was determined. The human BCDF and the human G-CSF used in this Example were the same as those used in the foregoing experiments.

As shown in Table 8 given below, the number of CFU-GM was significantly increased through the administration of (1) the human BCDF as compared with the control. Moreover, the number of CFU-GM was quite significantly increased through the simultaneous administration of (2) the human BCDF and the human G-CSF as compared with not only the control, but also those observed for the administration of the human BCDF alone and the human G-CSF alone.

**Table 8**

| | No. of CFU-GM/ml Peripheral Blood |
|---|---|
| Normal Mouse (free of administration of any chemotherapeutic agent) | 50 ± 10 |

| Mouse Treated (treated with chemotherapeutic agent) | |
|---|---|
| Control | 137 ± 45 |
| Administration of Human BCDF | 1433 ± 58* |
| Administration of Human G-CSF | 2667 ± 58* |
| Administration of Human BCDF + Human G-CSF | 42667 ± 9238*§ |
| Each data represented means ± standard deviaiton of 3 dishes × rate of dilution. | |

| | |
|---|---|
| *: This means that a significant difference is observed between each corresponding value and the control at a risk rate of 5%. | |
| § : This means that a significant difference is observed at a risk rate of 5% with respect to both the administration of the human BCDF alone and the administration of the human G-CSF alone. | |

### Example 9

### Survival Effect by Transplantation of Mononuclear cells In Peripheral Blood Which are Increased Through In Vivo Administration of (1) Human BCDF or (2) a Combination of Human BCDF and Human G-CSF

When mice (4 animals per group) were irradiated with X-rays at the lethal dose (10 Gy), all of the mice without any transplantation or to which mononuclear cells (contained in 0.1 ml of the peripheral blood) derived from the control mice were transplanted died within 14 days, while all of the mice transplanted, through the tail vein, with mononuclear cells (contained in 0.1 ml of the peripheral blood) derived from the mice to which both human BCDF and human G-CSF were administered survived. This clearly indicates that the peripheral blood derived from the mice to which both human BCDF and human G-CSF are administered can be used in stem cell transplantation.

In other experiments, when the peripheral blood mononuclear cells (contained in 0.4 ml of the peripheral blood) derived from the mice to which human BCDF was administered were transplanted to mice which were irradiated with X-rays at the lethal dose (10 Gy), all of the mice examined (5 animals) survived over not less than 60 days. This indicates that the peripheral blood derived from the mice to which human BCDF is administered can be used in stem cell transplantation.

In further experiments separately carried out, to mice (recipient mice; 8 animals per group) irradiated with X-rays at the lethal dose (10 Gy), there were transplanted the peripheral blood mononuclear cells (contained in 0.1 ml of the peripheral blood) derived from the mice (donor mice) to which 140 µg of human BCDF (10 µg/animal × 14 days) and/or 5.04 µg of human G-CSF (0.36 µg/animal × 14 days) were subcutaneously administered according to sustained administration through the tail vein. Incidentally, as a control, HSA (human serum albumin) was subcutaneously administered to mice, through sustained administration in an amount identical to that of the human BCDF.

As shown in Table 9 given below, all of the mice without any transplantation or to which mononuclear cells derived from the control mice were transplanted died within 14 days, while 6 animals transplanted with mononuclear cells derived from the mice to which human BCDF was administered survived over not less than 14 days and one animal survived over not less than 100 days. Seven out of 8 animals transplanted, through the tail vein, with mononuclear cells derived from the mice to which both human BCDF and human G-CSF were administered survived over not less than 100 days. The human BCDF and the human G-CSF used in Example 9 were the same as those used in the foregoing experiments.

**Table 9**

| | No. of Survived Recipient Mice | | |
|---|---|---|---|
| | 14 Days¹⁾ | 40 Days¹⁾ | 100 Days¹⁾ |
| X-ray Irradiated (no transplantation) | 0 | 0 | 0 |

| Donor Mice | | | |
|---|---|---|---|
| Control | 0 | 0 | 0 |
| Administration of Human BCDF | 6* | 3 | 1 |
| Administration of Human G-CSF | 4* | 4* | 3 |
| Administration of Human BCDF + Human G-CSF | 8* | 8* § | 7* § |
| Each data represented the number of survived mice out of 8 recipient mice examined. | | | |

| | | | |
|---|---|---|---|
| *: This means that a significant difference is observed between each corresponding value and the control at a risk rate of 5%. | | | |
| § : This means that a significant difference is observed at a risk rate of 5% with respect to both the administration of the human BCDF alone and the administration of the human G-CSF alone. | | | |
| 1) The time elapsed (Days) after the transplantation. | | | |

Sequence Listing

Sequence No.: 1

Length of Sequence: 184

Type of Sequence: amino acid

Topology: linear

Kind of Sequence: protein

Sequence:

Pro Val Pro Pro Gly Glu Asp Ser Lys Asp Val Ala Ala Pro His Arg
1               5                   10                  15
Gln Pro Leu Thr Ser Ser Glu Arg Ile Asp Lys Gln Ile Arg Tyr Ile
            20                  25                  30
Leu Asp Gly Ile Ser Ala Leu Arg Lys Glu Thr Cys Asn Lys Ser Asn
        35                  40                  45
Met Cys Glu Ser Ser Lys Glu Ala Leu Ala Glu Asn Asn Leu Asn Leu
    50                  55                  60
Pro Lys Met Ala Glu Lys Asp Gly Cys Phe Gln Ser Gly Phe Asn Glu
65                  70                  75                  80
Glu Thr Cys Leu Val Lys Ile Ile Thr Gly Leu Leu Glu Phe Glu Val
                85                  90                  95
Tyr Leu Glu Tyr Leu Gln Asn Arg Phe Glu Ser Ser Glu Glu Gln Ala
            100                 105                 110
Arg Ala Val Gln Met Ser Thr Lys Val Leu Ile Gln Phe Leu Gln Lys
        115                 120                 125
Lys Ala Lys Asn Leu Asp Ala Ile Thr Thr Pro Asp Pro Thr Thr Asn
    130                 135                 140
Ala Ser Leu Leu Thr Lys Leu Gln Ala Gln Asn Gln Trp Leu Gln Asp
145                 150                 155                 160

Met Thr Thr His Leu Ile Leu Arg Ser Phe Lys Glu Phe Leu Gln Ser
                165                 170                 175
Ser Leu Arg Ala Leu Arg Gln Met
            180

Sequence No.: 2
Length of Sequence: 185
Type of Sequence: amino acid
Topology: linear
Kind of Sequence: protein
Sequence:
Ala Pro Val Pro Pro Gly Glu Asp Ser Lys Asp Val Ala Ala Pro His
1               5                   10                  15
Arg Gln Pro Leu Thr Ser Ser Glu Arg Ile Asp Lys Gln Ile Arg Tyr
            20                  25                  30
Ile Leu Asp Gly Ile Ser Ala Leu Arg Lys Glu Thr Cys Asn Lys Ser
        35                  40                  45
Asn Met Cys Glu Ser Ser Lys Glu Ala Leu Ala Glu Asn Asn Leu Asn
    50                  55                  60
Leu Pro Lys Met Ala Glu Lys Asp Gly Cys Phe Gln Ser Gly Phe Asn
65                  70                  75                  80
Glu Glu Thr Cys Leu Val Lys Ile Ile Thr Gly Leu Leu Glu Phe Glu
                85                  90                  95
Val Tyr Leu Glu Tyr Leu Gln Asn Arg Phe Glu Ser Ser Glu Glu Gln
            100                 105                 110
Ala Arg Ala Val Gln Met Ser Thr Lys Val Leu Ile Gln Phe Leu Gln
        115                 120                 125
Lys Lys Ala Lys Asn Leu Asp Ala Ile Thr Thr Pro Asp Pro Thr Thr

    130                 135                 140
Asn Ala Ser Leu Leu Thr Lys Leu Gln Ala Gln Asn Gln Trp Leu Gln
145                 150                 155                 160
Asp Met Thr Thr His Leu Ile Leu Arg Ser Phe Lys Glu Phe Leu Gln
                165                 170                 175
Ser Ser Leu Arg Ala Leu Arg Gln Met
            180                 185

Sequence No.: 3
Length of Sequence: 177
Type of Sequence: amino acid
Topology: linear
Kind of Sequence: protein
Sequence:
Thr Pro Leu Gly Pro Ala Ser Ser Leu Pro Gln Ser Phe Leu Leu Lys
1               5                   10                  15
Cys Leu Glu Gln Val Arg Lys Ile Gln Gly Asp Gly Ala Ala Leu Gln
            20                  25                  30
Glu Lys Leu Val Ser Glu Cys Ala Thr Tyr Lys Leu Cys His Pro Glu
        35                  40                  45
Glu Leu Val Leu Leu Gly His Ser Leu Gly Ile Pro Trp Ala Pro Leu
    50                  55                  60
Ser Ser Cys Pro Ser Gln Ala Leu Gln Leu Ala Gly Cys Leu Ser Gln
65                  70                  75                  80
Leu His Ser Gly Leu Phe Leu Tyr Gln Gly Leu Leu Gln Ala Leu Glu
                85                  90                  95
Gly Ile Ser Pro Glu Leu Gly Pro Thr Leu Asp Thr Leu Gln Leu Asp
            100                 105                 110

Val Ala Asp Phe Ala Thr Thr Ile Trp Gln Gln Met Glu Glu Leu Gly
        115                 120                 125
Met Ala Pro Ala Leu Gln Pro Thr Gln Gly Ala Met Pro Ala Phe Ala
    130                 135                 140
Ser Ala Phe Gln Arg Arg Ala Gly Gly Val Leu Val Ala Ser His Leu
145                 150                 155                 160
Gln Ser Phe Leu Glu Val Ser Tyr Arg Val Leu Arg His Leu Ala Gln
                165                 170                 175
Pro

Sequence No.: 4
Length of Sequence: 174
Type of Sequence: amino acid
Topology: linear
Kind of Sequence: protein
Sequence:
Thr Pro Leu Gly Pro Ala Ser Ser Leu Pro Gln Ser Phe Leu Leu Lys
1               5                   10                  15
Cys Leu Glu Gln Val Arg Lys Ile Gln Gly Asp Gly Ala Ala Leu Gln
            20                  25                  30
Glu Lys Leu Cys Ala Thr Tyr Lys Leu Cys His Pro Glu Glu Leu Val
        35                  40                  45
Leu Leu Gly His Ser Leu Gly Ile Pro Trp Ala Pro Leu Ser Ser Cys
    50                  55                  60
Pro Ser Gln Ala Leu Gln Leu Ala Gly Cys Leu Ser Gln Leu His Ser
65                  70                  75                  80
Gly Leu Phe Leu Tyr Gln Gly Leu Leu Gln Ala Leu Glu Gly Ile Ser

                85                  90                  95
Pro Glu Leu Gly Pro Thr Leu Asp Thr Leu Gln Leu Asp Val Ala Asp
            100                 105                 110
Phe Ala Thr Thr Ile Trp Gln Gln Met Glu Glu Leu Gly Met Ala Pro
        115                 120                 125
Ala Leu Gln Pro Thr Gln Gly Ala Met Pro Ala Phe Ala Ser Ala Phe
    130                 135                 140
Gln Arg Arg Ala Gly Gly Val Leu Val Ala Ser His Leu Gln Ser Phe
145                 150                 155                 160
Leu Glu Val Ser Tyr Arg Val Leu Arg His Leu Ala Gln Pro
                165                 170

## Claims

1. A hemopoietic cell proliferation-promoting agent comprising human BCDF capable of promoting proliferation of hemopoietic cells through in vivo administration.

2. The hemopoietic cell proliferation-promoting agent of claim 1 wherein the hemopoietic cell is at least one member selected from the group consisting of leukocytes, platelets, hemopoietic progenitor cells and pluripotent hemopoietic stem cells.

3. The hemopoietic cell proliferation-promoting agent of claim 2 wherein the hemopoietic cell is a cell present in peripheral blood.

4. The hemopoietic cell proliferation-promoting agent of claim 3 wherein the hemopoietic cell is leukocyte present in peripheral blood.

5. The hemopoietic cell proliferation-promoting agent of claim 3 wherein the hemopoietic cell is platelet present in peripheral blood.

6. The hemopoietic cell proliferation-promoting agent of claim 3 wherein the hemopoietic cell is a hemopoietic progenitor cell present in peripheral blood.

7. The hemopoietic cell proliferation-promoting agent of claim 3 wherein the hemopoietic cell is a pluripotent hemopoietic stem cell present in peripheral blood.

8. The hemopoietic cell proliferation-promoting agent of claim 1 wherein the hemopoietic cell is a cell capable of being used in peripheral blood stem cell transplantation.

9. The hemopoietic cell proliferation-promoting agent of claim 1 wherein it further comprises human G-CSF.

10. The hemopoietic cell proliferation-promoting agent of claim 9 wherein the hemopoietic cell is at least one member selected from the group consisting of leukocytes, platelets, hemopoietic progenitor cells and pluripotent hemopoietic stem cells.

11. The hemopoietic cell proliferation-promoting agent of claim 10 wherein the hemopoietic cell is a cell present in peripheral blood.

12. The hemopoietic cell proliferation-promoting agent of claim 11 wherein the hemopoietic cell is leukocyte present in peripheral blood.

13. The hemopoietic cell proliferation-promoting agent of claim 11 wherein the hemopoietic cell is platelet present in peripheral blood.

14. The hemopoietic cell proliferation-promoting agent of claim 11 wherein the hemopoietic cell is a hemopoietic progenitor cell present in peripheral blood.

15. The hemopoietic cell proliferation-promoting agent of claim 11 wherein the hemopoietic cell is a pluripotent hemopoietic stem cell present in peripheral blood.

16. The hemopoietic cell proliferation-promoting agent of claim 9 wherein the hemopoietic cell is a cell capable of being used in peripheral blood stem cell transplantation.

17. A method for promoting proliferation of hemopoietic cells comprising the step of in vivo-administering human BCDF.

18. The method for promoting proliferation of hemopoietic cells according to claim 17 wherein the hemopoietic cell is at least one member selected from the group consisting of leukocytes, platelets, hemopoietic progenitor cells and pluripotent hemopoietic stem cells.

19. The method for promoting proliferation of hemopoietic cells according to claim 18 wherein the hemopoietic cell is a cell present in peripheral blood.

20. The method for promoting proliferation of hemopoietic cells according to claim 19 wherein the hemopoietic cell is leukocyte present in peripheral blood.

21. The method for promoting proliferation of hemopoietic cells according to claim 19 wherein the hemopoietic cell is platelet present in peripheral blood.

22. The method for promoting proliferation of hemopoietic cells according to claim 19 wherein the hemopoietic cell is a hemopoietic progenitor cell present in peripheral blood.

23. The method for promoting proliferation of hemopoietic cells according to claim 19 wherein the hemopoietic cell is a pluripotent hemopoietic stem cell present in peripheral blood.

24. The method for promoting proliferation of hemopoietic cells according to claim 17 wherein the hemopoietic cell is a cell capable of being used in peripheral blood stem cell transplantation.

25. The method for promoting proliferation of hemopoietic cells according to claim 17 wherein human G-CSF is simultaneously administered.

26. The method for promoting proliferation of hemopoietic cells according to claim 25 wherein the hemopoietic cell is at least one member selected from the group consisting of leukocytes, platelets, hemopoietic progenitor cells and pluripotent hemopoietic stem cells.

27. The method for promoting proliferation of hemopoietic cells according to claim 26 wherein the hemopoietic cell is a cell present in peripheral blood.

28. The method for promoting proliferation of hemopoietic cells according to claim 27 wherein the hemopoietic cell is leukocyte present in peripheral blood.

29. The method for promoting proliferation of hemopoietic cells according to claim 27 wherein the hemopoietic cell is platelet present in peripheral blood.

30. The method for promoting proliferation of hemopoietic cells according to claim 27 wherein the hemopoietic cell is a hemopoietic progenitor cell present in peripheral blood.

31. The method for promoting proliferation of hemopoietic cells according to claim 27 wherein the hemopoietic cell is a pluripotent hemopoietic stem cell present in peripheral blood.

32. The method for promoting proliferation of hemopoietic cells according to claim 25 wherein the hemopoietic cell is a cell capable of being used in peripheral blood stem cell transplantation.

33. A method for transplanting a peripheral blood stem cell comprising the steps of administering human BCDF to a normal person or a patient per se, collecting the peripheral blood from the person or patient and transplanting the peripheral blood to the patient.

34. The method for transplanting a peripheral blood stem cell according to claim 33 wherein human G-CSF is simultaneously administered to the normal person or the patient.

35. Use of human BCDF for promoting proliferation of hemopoietic cells by in vivo administration thereof.

36. The use of human BCDF according to claim 35 wherein the human BCDF is administered in combination with human G-CSF.

## Amended claims

### Amended claims under Art. 19.1 PCT

1. (Amended) A hemopoietic cell proliferation-promoting agent comprising human BCDF capable of promoting proliferation of hemopoietic cells present in peripheral blood through in vivo administration.

2. The hemopoietic cell proliferation-promoting agent of claim 1 wherein the hemopoietic cell is at least one member selected from the group consisting of leukocytes, platelets, hemopoietic progenitor cells and pluripotent hemopoietic stem cells.

3. (Cancelled)

4. (Amended) The hemopoietic cell proliferation-promoting agent of claim 1 wherein the hemopoietic cell is leukocyte.

5. (Amended) The hemopoietic cell proliferation-promoting agent of claim 1 wherein the hemopoietic cell is platelet.

6. (Amended) The hemopoietic cell proliferation-promoting agent of claim 1 wherein the hemopoietic cell is a hemopoietic progenitor cell.

7. (Amended) The hemopoietic cell proliferation-promoting agent of claim 1 wherein the hemopoietic cell is a pluripotent hemopoietic stem cell.

8. The hemopoietic cell proliferation-promoting agent of claim 1 wherein the hemopoietic cell is a cell capable of being used in peripheral blood stem cell transplantation.

9. The hemopoietic cell proliferation-promoting agent of claim 1 wherein it further comprises human G-CSF.

10. The hemopoietic cell proliferation-promoting agent of claim 9 wherein the hemopoietic cell is at least one member selected from the group consisting of leukocytes, platelets, hemopoietic progenitor cells and pluripotent hemopoietic stem cells.

11. (Cancelled)

12. (Amended) The hemopoietic cell proliferation-promoting agent of claim 9 wherein the hemopoietic cell is leukocyte.

13. (Amended) The hemopoietic cell proliferation-promoting agent of claim 9 wherein the hemopoietic cell is platelet.

14. (Amended) The hemopoietic cell proliferation-promoting agent of claim 9 wherein the hemopoietic cell is a hemopoietic progenitor cell.

15. (Amended) The hemopoietic cell proliferation-promoting agent of claim 9 wherein the hemopoietic cell is a pluripotent hemopoietic stem cell.

16. (Amended) The hemopoietic cell proliferation-promoting agent of claim 9 wherein the hemopoietic cell is a cell capable of being used in peripheral blood stem cell transplantation.

17. (Amended) A method for promoting proliferation of hemopoietic cells present in peripheral blood comprising the step of in vivo-administering human BCDF.

18. The method for promoting proliferation of hemopoietic cells according to claim 17 wherein the hemopoietic cell is at least one member selected from the group consisting of leukocytes, platelets, hemopoietic progenitor cells and pluripotent hemopoietic stem cells.

19. (Cancelled)

20. (Amended) The method for promoting proliferation of hemopoietic cells according to claim 19 wherein the hemopoietic cell is leukocyte.

21. (Amended) The method for promoting proliferation of hemopoietic cells according to claim 19 wherein the hemopoietic cell is platelet.

22. (Amended) The method for promoting proliferation of hemopoietic cells according to claim 19 wherein the hemopoietic cell is a hemopoietic progenitor cell.

23. (Amended) The method for promoting proliferation of hemopoietic cells according to claim 19 wherein the hemopoietic cell is a pluripotent hemopoietic stem cell.

24. The method for promoting proliferation of hemopoietic cells according to claim 17 wherein the hemopoietic cell is a cell capable of being used in peripheral blood stem cell transplantation.

25. The method for promoting proliferation of hemopoietic cells according to claim 17 wherein human G-CSF is simultaneously administered.

26. The method for promoting proliferation of hemopoietic cells according to claim 25 wherein the hemopoietic cell is at least one member selected from the group consisting of leukocytes, platelets, hemopoietic progenitor cells and pluripotent hemopoietic stem cells.

27. (Cancelled)

28. (Amended) The method for promoting proliferation of hemopoietic cells according to claim 27 wherein the hemopoietic cell is leukocyte.

29. (Amended) The method for promoting proliferation of hemopoietic cells according to claim 27 wherein the hemopoietic cell is platelet.

30. (Amended) The method for promoting proliferation of hemopoietic cells according to claim 27 wherein the hemopoietic cell is a hemopoietic progenitor cell.

31. (Amended) The method for promoting proliferation of hemopoietic cells according to claim 27 wherein the hemopoietic cell is a pluripotent hemopoietic stem cell.

32. The method for promoting proliferation of hemopoietic cells according to claim 25 wherein the hemopoietic cell is a cell capable of being used in peripheral blood stem cell transplantation.

33. A method for transplanting a peripheral blood stem cell comprising the steps of administering human BCDF to a normal person or a patient per se, collecting the peripheral blood from the person or patient and transplanting the peripheral blood to the patient.

34. The method for transplanting a peripheral blood stem cell according to claim 33 wherein human G-CSF is simultaneously administered to the normal person or the patient.

35. (Cancelled)

36. (Cancelled)

37. (Added) The hemopoietic cell proliferation-promoting agent of claim 2 wherein the hemopoietic cell is a cell capable of being used in peripheral blood stem cell transplantation.

38. (Added) The hemopoietic cell proliferation-promoting agent of claim 10 wherein the hemopoietic cell is a cell capable of being used in peripheral blood stem cell transplantation.

39. (Added) The method for promoting proliferation of hemopoietic cells according to claim 18 wherein the hemopoietic cell is a cell capable of being used in peripheral blood stem cell transplantation.
